# FASCICULE DE BREVET EUROPEEN

(11) **EP 3 365 465 B1**
(45) Date de publication et mention de la délivrance du brevet: **09.12.2020**
(21) Numéro de dépôt: 16809460.5
(22) Date de dépôt: 19.10.2016
(51) Int. Cl.: C12Q 1/6895, C12Q 1/04, G01N 33/569

(54) **PROCÉDÉ D'ANALYSE D'UN ÉCHANTILLON POUR LA PRÉSENCE DE LEVURES DE L'ESPÈCE BRETTANOMYCES BRUXELLENSIS RÉSISTANTES AUX SULFITES ET KIT POUR SA MISE EN OEUVRE**
VERFAHREN ZUR ANALYSE EINER PROBE ZUR DETEKTION DES VORHANDENSEINS VON SULFITRESISTENTEN HEFEN DER GATTUNG BRETTANOMYCES BRUXELLENSIS UND KIT ZUR DURCHFÜHRUNG DES VERFAHRENS
METHOD FOR ANALYSING A SAMPLE TO DETECT THE PRESENCE OF SULPHITE-RESISTANT YEASTS OF THE BRETTANOMYCES BRUXELLENSIS SPECIES AND KIT FOR IMPLEMENTING SAME

(30) Priorité: 20.10.2015 FR 1559975
(43) Date de publication de la demande: 29.08.2018
(73) Titulaire: Université de Bordeaux, 33000 Bordeaux (FR); Institut Polytechnique de Bordeaux, 33400 Talence (FR); École Nationale Supérieure des Sciences Agronomiques de Bordeaux-Aquitaine (Bordeaux Sciences Agro), 33170 Gradignan (FR)
(72) Inventeur: ALBERTIN, Warren, 33720 Podensac (FR); MASNEUF-POMAREDE, Isabelle, 33400 Talence (FR); PELTIER, Emilien, 33290 Parempuyre (FR)
(74) Mandataire: Ipside
(86) Numéro de dépôt international: PCT/FR2016/052701
(87) Numéro de publication internationale: WO 2017/068284

(56) Documents cités:
- EP-A1- 2 020 448
- Anonymous: "Preserve the Value Of Your Wine - Managing Brettanomyces Proactively Through On-site Analysis at the Winery", , 10 juillet 2015 (2015-07-10), XP055291980, Extrait de l'Internet: URL:http://invisiblesentinel.com/wp-conten t/uploads/2014/05/03110_IVS_vinoBRETT_web. pdf [extrait le 2016-07-28]
- CHRIS D. CURTIN ET AL: "De-Novo Assembly and Analysis of the Heterozygous Triploid Genome of the Wine Spoilage Yeast Dekkera bruxellensis AWRI1499", PLOS ONE, vol. 7, no. 3, 28 mars 2012 (2012-03-28), page e33840, XP055291868, DOI: 10.1371/journal.pone.0033840
- C. CURTIN ET AL: "Genotype-dependent sulphite tolerance of Australian Dekkera (Brettanomyces) bruxellensis wine isolates", LETTERS IN APPLIED MICROBIOLOGY, vol. 55, no. 1, 24 mai 2012 (2012-05-24), pages 56-61, XP055291873, GB ISSN: 0266-8254, DOI: 10.1111/j.1472-765X.2012.03257.x cité dans la demande
- ANTHONY R. BORNEMAN ET AL: "Insights into the Dekkera bruxellensis Genomic Landscape: Comparative Genomics Reveals Variations in Ploidy and Nutrient Utilisation Potential amongst Wine Isolates", PLOS GENETICS, vol. 10, no. 2, 13 février 2014 (2014-02-13), page e1004161, XP055291842, DOI: 10.1371/journal.pgen.1004161

### SEQUENCE LISTING

<110> UNIVERSITE DE BORDEAUX
   INSTITUT POLYTECHNIQUE DE BORDEAUX
   ECOLE NATIONALE SUPERIEURE DES SCIENCES AGRONOMIQUES DE
   BORDEAUX AQUITAINE BORDEAUX SCIENCES AGRO
<120> PROCÉDÉ D'ANALYSE D'UN ÉCHANTILLON POUR LA PRÉSENCE DE LEVURES DE L'ESPÈCE BRETTANOMYCES BRUXELLENSIS RÉSISTANTES AUX SULFITES ET KIT POUR SA MISE EN OEUVRE
<130> 31630 WO
<150> FR 1559975
   <151> 2015-10-20
<160> 8
<170> PatentIn version 3.5
<210> 1
   <211> 1020
   <212> PRT
   <213> Brettanomyces bruxellensis
<220>
   <221> misc_feature
   <222> (347)..(347)
   <223> Xaa can be any naturally occurring amino acid
<220>
   <221> misc_feature
   <222> (550)..(550)
   <223> Xaa can be any naturally occurring amino acid
<220>
   <221> misc_feature
   <222> (562)..(562)
   <223> Xaa can be any naturally occurring amino acid
<220>
   <221> misc_feature
   <222> (600)..(600)
   <223> Xaa can be any naturally occurring amino acid
<220>
   <221> misc_feature
   <222> (612)..(612)
   <223> Xaa can be any naturally occurring amino acid
<220>
   <221> misc_feature
   <222> (617)..(617)
   <223> Xaa can be any naturally occurring amino acid
<220>
   <221> misc_feature
   <222> (695)..(695)
   <223> Xaa can be any naturally occurring amino acid
<220>
   <221> misc_feature
   <222> (770)..(770)
   <223> Xaa can be any naturally occurring amino acid
<220>
   <221> misc_feature
   <222> (779)..(779)
   <223> Xaa can be any naturally occurring amino acid
<220>
   <221> misc_feature
   <222> (889)..(889)
   <223> Xaa can be any naturally occurring amino acid
<220>
   <221> misc_feature
   <222> (946)..(946)
   <223> Xaa can be any naturally occurring amino acid
<220>
   <221> misc_feature
   <222> (980)..(980)
   <223> Xaa can be any naturally occurring amino acid
<400> 1
   Met Arg Leu Met Pro Gln Lys Lys Arg Asp Lys Lys Ser Leu Ser Gly
   1               5                   10                  15
   Val Glu His Val Asn Pro Asp Asn Gly Gly Asp Asp Ser Gly Glu Gly
               20                  25                  30
   Asp Gly Asn Asx Asp Asn Ala Asp Asp Asp Asp Asp Asp Asp Asp Val
           35                  40                  45
   Asn Met Thr Met Met Asn Asp Glu Ser Tyr Tyr Ala Asp Gly Ser Val
       50                  55                  60
   Glu Glu Gly Arg Val Leu Asn Ser Ser Val Asp Asn Asp Leu Tyr Leu
   65                  70                  75                  80
   Lys Pro Gly Phe Gly Ser Ala Arg Asn Leu Cys Ile Thr Leu Arg Tyr
                   85                  90                  95
   Leu Leu Tyr Glu Gln Ala Val Asp Tyr Phe Tyr Asp Glu Phe Asn Asn
               100                 105                 110
   Arg Asp Ser Lys Phe His Asp Phe Glu Leu Leu Asp Glu Gln His Asp
           115                 120                 125
   Ala Asn Lys Asp Glu Thr Ser Ala Lys Asn Leu His Asp Leu Ser Glu
       130                 135                 140
   Ser Ser Glu Asp Asn Val Ser Asn Thr Glu Asn Asp Ala Glu Asp Asp
   145                 150                 155                 160
   Asn Tyr Asp Asp Asp Ser Pro Lys Ile Glu Arg Gly Asp Ala Ser Pro
                   165                 170                 175
   Glu Asp Asp Asp Tyr Asp Asp Asp Asp Asp Asp Asp Asp Asp Asp Lys
               180                 185                 190
   Gly Asp Glu Asp Asn Thr Lys Asp Thr Asn Asn Asp Asn Ser Val Lys
           195                 200                 205
   Val Ile Ser Thr Gly Ile Pro Val Gly Val Asn Asp Lys Gly Ser Glu
       210                 215                 220
   Glu Asp Lys Ser Leu Asn Asp Glu Asn Ser Lys Val Asp Asp Glu Asn
   225                 230                 235                 240
   Asp Val Ser Pro Pro Lys Tyr Ile Glu Pro Gly Ser His Gly Glu Met
                   245                 250                 255
   Ile Leu Lys Val Pro Glu Ser Ala Ile Leu Ser Lys Pro Ser Tyr Pro
               260                 265                 270
   Ser Leu Pro Asn Ser Phe Ser Pro Ala Asp Glu Ala Ile Gly Lys Val
           275                 280                 285
   Val His Pro Ile Leu Ser Thr Ser Ser Ala Ile Glu Ser Ser Leu Glu
       290                 295                 300
   Lys Gln Asn Glu Leu Lys Leu Ile Lys Asn Phe Asn Lys Ile Tyr His
   305                 310                 315                 320
   Gly Phe Asp Asp Asp Leu Gln Asn Ile Val Lys Arg Arg Lys Leu Glu
                   325                 330                 335
   Lys Ser Asn Gln Gln Leu Gly Leu Glu Ser Xaa Glu Ser Asn Ser Ser
               340                 345                 350
   Lys Thr Asp Gly Lys Leu Asp Gln Ser Val Ser Lys Leu Met Ser Leu
           355                 360                 365
   Gly Gly Ala Ala Asn Leu Ser Leu Lys Asn Leu Leu Gly Arg Ile Glu
       370                 375                 380
   Asp Asn Arg Ser Lys Leu Asn Ile Thr Asp Ile Glu Leu Lys Asn Leu
   385                 390                 395                 400
   Ile Met Asp Val Arg Lys Asn Arg Ser Lys Trp Ala Asn Tyr Asn Arg
   405 410 415
   Ile Gly Gln Glu Glu Leu Tyr Glu Ala Cys Glu Lys Val Val Thr Glu
   420 425 430
   Leu Arg Gly Tyr Thr Glu His Ser Thr Pro Phe Leu Asn Arg Val Ser
   435 440 445
   Lys Arg Glu Ala Pro Asn Tyr Tyr Glu Ile Ile Lys Lys Pro Met Asp
   450 455 460
   Leu Asn Thr Val Met Arg Lys Leu Arg Asn Phe Gln Tyr Arg Ser Lys
   465 470 475 480
   Lys Gln Phe Val Asp Asp Leu Met Leu Ile Trp Gln Asn Cys Leu Thr
   485 490 495
   Tyr Asn Thr Asp Pro Lys His Tyr Leu Arg Val Asp Ala Ile Ala Met
   500 505 510
   Gln Lys Lys Thr Gln Ser Leu Ile Pro Leu Ile Pro Asp Ile Val Val
   515 520 525
   Arg Asp Arg Ala Glu Val Glu Lys Glu Ala Ala Leu Ala Ala Lys Gln
   530 535 540
   Asp Lys Glu Glu Gln Xaa Ser Glu Gln Glu Thr Glu Asn Ala Thr Arg
   545 550 555 560
   Ser Xaa Arg Ala Val Gly Ala Val Gly Thr Gly Ser Lys Gln Thr Ala
   565 570 575
   Lys Lys His Arg Lys Gly Pro Thr Gln Glu Asp Asn Gln Glu Glu Val
   580 585 590
   Glu Ser Lys Glu Lys Thr Ala Xaa Leu Asp Ser Lys Thr Glu Glu Glu
   595 600 605
   Thr Thr Thr Xaa Ser Ala Thr Pro Xaa Gly Thr Asp Thr Pro Ser Thr
   610 615 620
   Ala His Asp Glu Lys His Leu Pro Ile Asn Leu Thr Lys Ser Val Ser
   625 630 635 640
   Lys Val Asp Ser Met Thr Pro Ala Thr Ala Ser Gly Ala Ser Gly Thr
   645 650 655
   Pro Pro Gln Asn Ile Asp Glu Glu Asn Asp Glu Leu Asp Glu Ala Leu
               660                 665                 670
   Glu Glu Leu Arg Thr Glu Arg Asn Glu Asp Asp Asp Asp Asn Asp Val
           675                 680                 685
   Ile Glu Asp Leu Glu Ala Xaa Thr Trp Lys Thr Leu Thr Ala Asn Thr
       690                 695                 700
   Arg Tyr Lys Leu Cys Glu Ala Arg Gly Lys Met Phe Lys Gly Asn Lys
   705                 710                 715                 720
   Ile Asp Pro Asn Val Glu Ser Leu Tyr Arg Asp Glu Asn Gln Met Ala
                   725                 730                 735
   Asn Phe Met Asn Tyr Leu Thr Asp Asn Gly Asp Leu Val Ile His Lys
               740                 745                 750
   Asn Arg Tyr Phe Asp Glu His Asp Asp Pro Tyr Leu Ile Glu Tyr Asp
           755                 760                 765
   Val Xaa Gly Gly Val Pro Pro Ile Lys Phe Xaa Gly Val Asn Phe Glu
       770                 775                 780
   Gln Glu Glu Asn Ala Met Met Glu Lys Leu Leu Gly Lys Gly Glu Thr
   785                 790                 795                 800
   Leu Asp Ser Leu Pro Asp Ser Leu Leu Lys Val Lys Ile Lys Gly Ser
                   805                 810                 815
   Asn Ser Val Ile Met Asp Asn Ile Lys Thr Met Gln Gln Ile Arg Lys
               820                 825                 830
   Thr Cys Phe Arg Ile Asn Leu Ile Arg Gln Met Gln Thr Ser Gln Phe
           835                 840                 845
   Val His Arg Ser Gln Met Ser Ala Pro Asp Ile Lys Glu Ile Asn Phe
       850                 855                 860
   Asn Asp Ile Asp Pro Leu Ser Lys Leu Pro Thr Arg Asp Ser Leu Asn
   865                 870                 875                 880
   Gly Glu Val Ala Phe Arg Ser Leu Xaa Lys Ser Ile Ser Ala Ile Leu
                   885                 890                 895
   Met Ala Thr Gly Phe Glu Lys Thr Ser Pro Phe Cys Ala Ser Leu Met
               900                 905                 910
   Thr Gln Leu Ala Glu Glu His Leu Gly Asn Leu Ala Lys Ser Met Lys
           915                 920                 925
   Leu His Glu Glu Ser Asn Ser Ile Asn Lys Met Asn Ile Arg Gly Lys
       930                 935                 940
   Ser Xaa Thr Asn Ile Lys Asn Ile Val Gln Met Ser Leu Glu Glu Asn
   945                 950                 955                 960
   Gly Ile Asp Lys Pro Asp Ile Ile Tyr Thr Tyr Tyr Lys Glu Tyr Leu
                   965                 970                 975
   Ala Lys Arg Xaa Lys Lys Leu Lys Asp Leu Lys Thr Ala Leu Glu Glu
               980                 985                 990
   Phe Leu Lys Glu Leu Leu Arg Pro  Gly Leu Gln Asp Ile  Asn Glu Thr
           995                 1000                 1005
   Gln Phe  Thr Asp Asn Ser Asp  Gln Phe Tyr Asp Trp
       1010                 1015                 1020
<210> 2
   <211> 5063
   <212> DNA
   <213> Brettanomyces bruxellensis
<400> 2
   cttattattg gtacaagttt atatatcata aatacaaaca cggattaaag aaataaaaga        60
   aataaaaatg aaaaaaaaga aacccatcac acgcctaaaa aaaaggataa gycatgactc       120
   tatcacgcrt cacctatgtc ttccgcctta agttttatca gacgaaatat ctttatcatc       180
   aatatcaccc tgtgatgcat ccgtgaaaat atctttatcg tcgcctttct cytcatyctc       240
   atcgttatca tcttcgccaw cttcattgat tgtrtcaagt atrccatttg aatctttctk       300
   ytccgtttca acgctttcct trtgatcgtt atygagtgaa tcatcattag caacttttct       360
   aattacagaa tmtccagcat tttctttcga tacctttcca tcggtcacac ttgttgtcgc       420
   gtccaattgg ccggaactak atgacttttc ggtatttrtt gtttcwtgcg mttgcgtctt       480
   ctycttttct tctgctaccc atttgtcttg atcgtcaata aagaamgaag tagcaatcag      540
   tttcttcttt atctgcgtaa tctttccatt aggaggaatt tttggtctat tattcctttg       600
   tttaatcggt agatcatcat cctcaataat tctcaaatca ttcaaatctt tatattcaac       660
   aaacttttca tttggatctg cagtaccrtt rgcaatatgc tgctggtgtc ttcgtaattg       720
   cttggcrtac atagtttcac ttttcgtaag taatcgttca taatagtgct taaggacrcc       780
   aatttgtttt ggaagatctt tcttccgaag ctttggaaac wtgaactctt tgaagtcatc       840
   atacttctct ttcyttctct tattgttcaa ytggttaaac tgatacgaaa gacgcgagtg 900
   yaataaatgc aacggaacac tagcwgaaag aatwccaaat tctttatcca atcccagttc 960
   tctgaaacca aagaaatcat ccccaatctc ctcsgaaaat tcaccagtca taaaactgat 1020
   cagaattatc agtgaactgy gtttcgttta tatcttgtaa tccaggtctt aacaattcct 1080
   tcaagaattc ttccagagca gtcttgagat ctttgagttt cttgytcctt ttagcaagat 1140
   attccttgta atacgtgtat attatatcyg gcttatcaat gccattttcc tcaagagaca 1200
   tttggacaat atttttgata ttcgttgrag acttacccct aatattcatc ttgttaatgg 1260
   arttggactc ttcatgcaat ttcatcgatt ttgcragatt tccaagatgc tcttccgcaa 1320
   gttgagtcat taaagaggca caaaawggac ttgttttttc aaatccrgtr gccatgagta 1380
   ttgcrgaaat agacttcygt aacgatctga aggcaacttc accattcaac gaatcyctgg 1440
   ttgggagctt tgagagcgga tcaatatcat tgaaattaat ttctttgatr tctggtgccg 1500
   acatttgcga ccggtgcaca aattgagagg tttgcatctg tctgataaga ttgattctaa 1560
   aacatgtctt tcgaatctgt tgcatggtct taatattatc cataataaca ctgttggagc 1620
   ccttgatctt cactttcagc aaactgtctg gaagagaatc taaagtttca cctttgccca 1680
   gtagcttttc catcatggca ttttcctcct gctcaaaatt cacacctytr aacttaatgg 1740
   gcggcactcc accyytaacr tcatattcta tcaaatasgg atcatcrtgy tcatcaaagt 1800
   atctattctt gtgaatcacc aaatcgccgt tatccgtaag atagttcatg aaattggcca 1860
   tctgattttc gtctctatag agcgactcca catttggatc aattttgttt cctttaaaca 1920
   tcttacctct rgcctcacat aacttatayc trgtrttygc agtcaaagtt ttccacgtgg 1980
   magcctctaa atcctcaatt acatcattat cgtcatcatc ttcattcctt tctgttcgaa 2040
   gttcctccaa agcttcgtct agttcatcgt tttcttcatc aatattttga ggtggagttc 2100
   cagaagcacc ggatgctgtt gcaggagtca tggaatcgac cttggataca cttttcgtca 2160
   ggtttattgg taaatgcttt tcatcatgyg cagttgatgg rgtatctgtt cccrctggtg 2220
   tagcagaaay cgttgtcgtt tcttcttcag tctttgaatc taaagyygcy gtcttttctt 2280
   tagattctac ttcttcctga ttgtcttcct gggttggacc ttttcgatgc tttttggccg 2340
   tttgcttact tccagttcca acagccccaa cagccctacy agatcttgta gcattctctg 2400
   tttcctgctc agawtcttgt tcctccttat cctgttttgc agccaatgca gcttcttttt 2460
   ccacttcagc tctatctctc accactatat ccggaatcaa cggtatcaat gactgygttt 2520
   tyttctgcat agctattgca tcaacacgaa ggtaatgctt tggatccgtg ttataagtca 2580
   aacagttttg ccaaattaac atcaaatcat cyacaaactg ctttttgctt ctatattgga 2640
   aatttctcag ctttctcatt actgtattca agtccattgg cttctttatg atttcataat 2700
   aattaggagc ttccctttta gagaccctgt ttagaaatgg cgtggaatgc tcagtataac 2760
   ctctcaactc agtgacaact ttctcrcagg cttcataaag ctcttcttgc ccaattctat 2820
   tgtagtttgc ccattttgat ctattctttc taacatccat tatcaagtty ttgagttcta 2880
   tgtctgtaat attaagcttg ctyctgttat cctctattct tcctaataaa ttttttaaag 2940
   aaaggttrgc ggctcccccw agactcataa gcttactmac actttgatca agtttkccat 3000
   ccgttttact tgaattcgat tcayctgact caagccccaa ttgctgatta ctyttctcaa 3060
   gcttccgrcg cttgactata ttttgtagat catcatcaaa accatgatat attttgttaa 3120
   agttcttaat caactttagc tcattctgct tctcaagact tgattcaata gcagacgatg 3180
   tgctgagaat tggatgaacg acttttccaa ttgcttcatc tgctggtgaa aacgagttcg 3240
   gaagacttgg ataacttggc ttggataata tcgcactttc cggaactttt aatatcattt 3300
   caccatgtga acctggttca atatatttag gaggagacac atcattttcg tcatctactt 3360
   tcgaattttc atcatttaag cttttatctt cctccgatcc tttatcatta actccgactg 3420
   ggattcctgt agaaataacc ttaactgaat tatcattatt agtgtccttt gtattatcct 3480
   catcaccctt atcatcatca tcatcatcat catcatcatc atagtcatca tcttctggag 3540
   aagcatcacc acgttcaatt tttggactgt cgtcatcata attatcatct tctgcatcat 3600
   tctcagtatt gcttacattg tcttcagagg attctgacaa atcgtgcaaa ttcttcgcag 3660
   atgtttcatc tttattagca tcatgctgtt catccaacaa ctcaaaatca tgaaacttgc 3720
   tatctctgtt attaaactcg tcataaaaat aatcaacagc ctgttcataa agaagatatc 3780
   gcaatgttat gcaaagattt cgtgcrgaac caaatccagg cttcaagtat aaatcattgt 3840
   caactgaact gttgagaaca cgmccttctt caacactscc atcagcataa tatgattcat 3900
   cattcatcat agtcatattg acatcatcat catcatcatc atcatcagca ttatcatyat 3960
   taccatcacc ttcaccacta tcatcaccac cattatccgg atttacatgt tcaacaccgg 4020
   acaaggactt cttatcgcgt tttttttgag gcatcagtcg cattatcatt caaaaattga 4080
   acaaaaagat tacagtccat taatgcatcc catgctgcaa gcttttgctc atctacttca 4140
   ctctttatag attgatcatc agaggatytc aaaatgaagc taaggaggtt aaacaattgc 4200
   ggactaaggt aacaattaaa agaatccttc attgaagagt ttgacggtca accgtcgcag 4260
   caacttctta ttatttcctt tgaatgcctc gagtttataa ccggccatta ttttggtctg 4320
   ttgaacaaaa aacaattatc caaatacaga tctgaaatat tttaagttga caccaaacga 4380
   aattaaaaat tcacccttta actgcaacgt tcttccagaa aagatacccg ggccaaggta 4440
   gctaatatat gtgaaatatg attactgtca aggaacacta atggatgaat agtgaaatgt 4500
   gatyctggaa gaactttgta gcctaactat aatcatgcag acgtatgagt tacttaaaaa 4560
   aaaaaawata ratraataaa tcgaatataa tataaaaaty gasaacaaaa ctactcacct 4620
   ctrggcacgc cgatcgacgt agtcyggkyt ttattttatt ttcrgargct attctataca     4680
   tattttttgc taatcgagga taaatcatgt gcgtataatr tgggcactag gyaaactgta     4740
   ataaytctac cgttttatat attttctttc aaaagtatak aagtttccta ctttcggrca     4800
   attmtttgtt tmttggatcr agagtttaag raaggaatac cctrttcaaa agracryagt     4860
   satcaattta aacgaatata ttggttagac ctaggaaaga gagctaggaa tagagaaagt     4920
   catataaaat gccagatttt gtggataaga catttgatgc agtatattca gtgtatcaga     4980
   cagcagtggg tgctctgaat ggagcatatg ataaggccaa ggatgctggg caaagcatta     5040
   tggaatatac aaaaaatagt gcc                                             5063
<210> 3
   <211> 21
   <212> DNA
   <213> Brettanomyces bruxellensis
<400> 3
   tcttcctccg atcctttatc a 21
<210> 4
   <211> 21
   <212> DNA
   <213> Brettanomyces bruxellensis
<400> 4
   ttgcacgatt tgtcagaatc c 21
<210> 5
   <211> 25
   <212> DNA
   <213> Brettanomyces bruxellensis
<400> 5
   agaagttgaa cggccgcatt tgcac 25
<210> 6
   <211> 25
   <212> DNA
   <213> Brettanomyces bruxellensis
<400> 6
   aggattgttg acactctcgc cgagg 25
<210> 7
   <211> 263
   <212> DNA
   <213> Brettanomyces bruxellensis
<400> 7
   tcttcctccg atcctttatc attaactccg actgggattc ctgtagaaat aaccttaact      60
   gaattatcat tattagtgtc ctttgtatta tcctcatcac ccttatcatc atcatcatca     120
   tcatcatcat catcatagtc atcatcttct ggagaagcat caccacgttc aatttttgga     180
   ctgtcgtcat cataattatc atcttctgca tcattctcag tattgcttac attgtcttca     240
   gaggattctg acaaatcgtg caa                                             263
<210> 8
   <211> 39
   <212> DNA
   <213> Brettanomyces bruxellensis
<400> 8
   acgacgttgt aaaacgactc ttcctccgat cctttatca 39

## Description

La présente invention concerne un procédé d'analyse d'un échantillon, notamment d'un échantillon de vin, pour la présence éventuelle de levures de l'espèce *Brettanomyces bruxellensis* résistantes aux sulfites, ainsi qu'un kit pour la mise en œuvre d'un tel procédé. L'invention concerne également l'utilisation, pour l'analyse de la présence éventuelle, dans un échantillon, de levures de l'espèce *Brettanomyces bruxellensis* résistantes aux sulfites, d'un couple d'amorces apte à amplifier une séquence nucléotidique spécifique des levures appartenant au groupe génétique des levures triploïdes œnologiques de l'espèce *Brettanomyces bruxellensis.*

Les levures de l'espèce *Brettanomyces bruxellensis* (également nommée *Dekkera bruxellensis*) constituent une des sources d'altération majeures des boissons fermentées, telles que le vin, la bière, le cidre, etc., et, plus particulièrement, la première cause de contamination des vins. Ces levures produisent en particulier des quantités importantes de phénols volatils, qui confèrent au vin un goût et une odeur désagréables, dont la description couramment utilisée est « odeurs d'écurie » ou « sueur de cheval ». Cette contamination peut être la cause de pertes économiques importantes, surtout lorsqu'elle touche les vins rouges élevés en fûts de bois. L'altération par *Brettanomyces bruxellensis* touche en effet environ 25 % des vins rouges, et elle entraîne un rejet quasi-systématique des vins par les consommateurs.

Les praticiens chargés de l'élaboration des vins disposent à l'heure actuelle de plusieurs méthodes pour lutter contre l'altération des vins par *Brettanomyces bruxellensis.*

Parmi ces méthodes, on peut citer les méthodes de prédiction de la présence de ces levures dans le vin, au moyen de logiciels tels que ceux commercialisés sous les noms de Bret'Less® et Brett Scoring®, qui évaluent, pour un vin donné, le risque de contamination en fonction de la composition des moûts et du vin, et des pratiques œnologiques sur le lieu d'élaboration du vin ; les méthodes de détection quantitatives ou semi-quantitatives de la présence de telles levures, notamment par étalements sur milieu sélectif de cette espèce, réaction de polymérisation en chaîne, ou cytométrie de flux, l'outil semi-quantitatif Veriflow® Brett, qui permet de préciser les niveaux de population de *Brettanomyces bruxellensis* dans un échantillon, pouvant être cité en particulier ; ou encore les méthodes curatives, dont le but est d'éliminer les phénols volatils produits, par exemple au moyen d'adsorbants tels que des écorces de levure, des charbons actifs, etc. Le document EP 2 020 448 A1 décrit un procédé de détection et de quantification de levures du genre *Brettanomyces* dans un échantillon. Aucune de ces méthodes ne s'avère cependant réellement satisfaisante. Les méthodes curatives, par exemple, ont généralement un impact organoleptique sur les vins traités.

Un autre type de méthodes couramment mises en œuvre pour se prémunir de la présence de levures de l'espèce *Brettanomyces bruxellensis* dans le vin sont les méthodes de lutte contre ces levures. Parmi elles, on peut citer la filtration, les traitements au chitosane, etc. Ces méthodes présentent cependant les inconvénients d'une mise en œuvre complexe, et de nécessiter des investissements coûteux, et elles ont souvent un impact négatif sur la qualité organoleptique des vins.

La méthode de lutte contre les levures de l'espèce *Brettanomyces bruxellensis* la plus répandue, car la moins contraignante à mettre en œuvre, est la méthode connue sous le nom de sulfitage, qui consiste à ajouter du dioxyde de soufre dans le vin, de sorte à tirer notamment profit des propriétés antioxydantes et antimicrobiennes du soufre. Cette méthode s'avère cependant inefficace lorsque les souches de *Brettanomyces bruxellensis* présentes dans le vin sont résistantes aux sulfites. Il a en effet été montré par l'art antérieur, comme notamment décrit dans la publication de Curtin et al., 2012, dans Letters in Applied Microbiology, 55: 56-61, que chez l'espèce *Brettanomyces bruxellensis* le trait phénotypique de résistance aux sulfites est souche-dépendant. La publication de Curtin et al., dans PLOS ONE, vol. 7, no. 3, 2012, décrit l'organisation génomique et la caractérisation fonctionnelle des gènes d'une souche triploïde particulière de *Dekkera bruxellensis.* Cette souche contient notamment un gène codant pour une protéine conférant la tolérance aux sulfites.

La mise en œuvre de la méthode de sulfitage s'avère en outre risquée car l'utilisation systématique de dioxyde de soufre à forte dose favorise l'émergence de nouvelles souches résistantes aux sulfites. Par exemple, il a été rapporté, dans la publication de Curtin et al., 2012, ci-dessus mentionnée, qu'en Australie, après une dizaine d'années d'usage intensif du sulfitage pour lutter contre *Brettanomyces bruxellensis,* 85 % des isolats sont aujourd'hui résistants aux sulfites. Par ailleurs, depuis quelques années, la politique générale en agriculture a pour objectif la diminution des intrants. En œnologie, cela se traduit par une forte pression sociétale pour la réduction des doses de sulfites employées, associée à une législation de plus en plus restrictive.

Ainsi, il existe à l'heure actuelle un besoin pour une méthode permettant la mise en place d'une stratégie d'utilisation raisonnée des sulfites pour le sulfitage du vin, c'est-à-dire une utilisation ciblée aux seuls vins contenant des levures *Brettanomyces bruxellensis* qui sont sensibles aux sulfites, pour lesquels le sulfitage présente une utilité.

A l'effet de proposer une telle méthode, diverses études ont été menées par l'art antérieur pour tenter de déterminer une relation entre le génotype des levures de l'espèce *Brettanomyces bruxellensis,* et leurs traits phénotypiques, et plus particulièrement leur capacité de résistance aux sulfites. C'est le cas par exemple de l'étude décrite dans la publication de Curtin et al., 2012, ci-dessus mentionnée, qui vise à identifier, à partir de nombreux isolats de *Brettanomyces bruxellensis,* une relation éventuelle entre le phénotype de résistance aux sulfites et des marqueurs génotypiques de ces levures.

Aucune étude n'a cependant permis à ce jour d'identifier un lien entre trait phénotypique de résistance aux sulfites et trait génotypique chez *Brettanomyces bruxellensis.*

La présente invention vise ainsi à proposer un procédé permettant de détecter de manière fiable, dans un échantillon susceptible de contenir des levures de l'espèce *Brettanomyces bruxellensis,* en particulier un échantillon d'un vin, la présence de levures résistantes aux sulfites, et le cas échéant leur proportion par rapport à la population totale de levures de l'espèce *Brettanomyces bruxellensis* présentes, de sorte à pouvoir éviter, ultérieurement, de traiter par sulfitage des vins dont on saurait alors qu'ils contiennent des levures résistantes aux sulfites, pour lesquels un tel traitement par sulfitage s'avérerait inefficace.

Un objectif supplémentaire de l'invention est que ce procédé soit simple et rapide à mettre en œuvre, et qui plus est à coût réduit et au moyen de dispositifs couramment présents dans les laboratoires d'analyse œnologique.

A l'origine de la présente invention, il a été découvert par les présents inventeurs que, de manière tout à fait inattendue, alors que les travaux de l'art antérieur suggéraient une relation d'interdépendance entre résistance aux sulfites et génotype des levures, la résistance aux sulfites des levures de l'espèce *Brettanomyces bruxellensis* est liée non pas à un génotype particulier de ces levures, mais à leur ploïdie, c'est-à-dire non pas à la qualité des gènes de ces levures, mais au nombre d'exemplaires de chromosomes qu'elles présentent. Plus particulièrement, il a été découvert par les présents inventeurs que parmi les trois groupes génétiques de l'espèce *Brettanomyces bruxellensis* décrits à ce jour, c'est-à-dire le groupe diploïde, le groupe triploïde œnologique et le groupe triploïde de bière, les souches de levures du groupe génétique triploïde œnologique, et elles seules, sont résistantes aux sulfites. Ainsi, la présence, dans un échantillon, notamment un échantillon dont la composition en microorganismes est représentative de celle d'un vin, de levures de l'espèce *Brettanomyces bruxellensis* appartenant au groupe génétique des levures triploïdes œnologiques, est indicative d'une résistance aux sulfites de levures contenues dans cet échantillon, alors que l'absence de telles levures est indicative d'une sensibilité aux sulfites des levures de cet échantillon. C'est dans ce dernier cas particulier que le traitement par sulfitage s'avère d'intérêt.

On entend dans la présente description, par groupe génétique des levures triploïdes œnologiques, de manière classique en elle-même, le groupe des levures triploïdes dont le substrat est majoritairement le vin, en particulier le vin rouge, le jus de raisin ou le raisin, ou le matériel vitivinicole. Par groupe génétique des levures triploïdes de bière, on entend le groupe des levures triploïdes dont le substrat est majoritairement la bière, ainsi que d'autres boissons fermentées telles que le cidre, la kombucha, la téquila, etc. Des exemples de souches appartenant à ces groupes, avec, pour chacune, le substrat associé, et la collection dans laquelle elle est conservée, et auprès de laquelle il est possible de se la procurer, sont indiquées dans la publication d'Albertin et al., 2014, dans Food Microbiology, 42: 188-195.

Ainsi, selon un premier aspect, il est proposé selon la présente invention un procédé d'analyse d'un échantillon, notamment d'une fraction d'un milieu liquide tel qu'une boisson fermentée, et en particulier un vin, ou déposé sur un support solide ou semi-solide, pour la présence éventuelle de levures de l'espèce *Brettanomyces bruxellensis* résistantes aux sulfites, tel que défini dans la revendication 1. Selon ce procédé :
- on procède à une étape de détection de la présence éventuelle, dans l'échantillon, de levures de l'espèce *Brettanomyces bruxellensis* appartenant au groupe génétique des levures triploïdes œnologiques,
- et, si on détecte une telle présence de levures de l'espèce *Brettanomyces bruxellensis* appartenant au groupe génétique des levures triploïdes œnologiques, on déduit que l'échantillon contient des levures de l'espèce *Brettanomyces bruxellensis* résistantes aux sulfites.

Au contraire, si on ne détecte aucune telle présence de levures de l'espèce *Brettanomyces bruxellensis* appartenant au groupe génétique des levures triploïdes œnologiques, on déduit, si l'échantillon contient des levures de l'espèce *Brettanomyces bruxellensis,* que ces levures sont sensibles aux sulfites.

L'étape de détection comprend la détection, pour des levures contenues dans l'échantillon, notamment dans un échantillon de vin, d'un marqueur moléculaire spécifique des levures de l'espèce *Brettanomyces bruxellensis* du groupe génétique des levures triploïdes œnologiques.

Par levures résistantes aux sulfites, on entend selon la présente invention des levures présentant la capacité de croitre en présence d'une concentration de sulfite(s) correspondant à la pratique en œnologie, c'est-à-dire de 0,4 mg/L de dioxyde de soufre moléculaire (H₂SO₃) dans le milieu. La sensibilité aux sulfites correspond quant à elle à l'incapacité des levures de croitre en présence d'une concentration de dioxyde de soufre moléculaire correspondant à une quantité supérieure ou égale à 0,4 mg/L dans le milieu.

Par le terme sulfites, on désigne la fraction active moléculaire du dioxyde de soufre présente dans un milieu donné. Cette fraction active peut notamment résulter de l'addition dans le milieu, notamment dans le jus de raisin ou le vin, de sels ou esters de l'acide sulfureux, tels que l'anhydride sulfureux, le sulfite de sodium, le bisulfite de sodium, le métabisulfite de sodium, le métabisulfite de potassium, le sulfite de potassium, le sulfite de calcium, le bisulfite de calcium, le bisulfite de potassium, etc., ainsi que de dioxyde de soufre.

A l'issue de la mise en œuvre du procédé d'analyse selon l'invention, le résultat du diagnostic ainsi obtenu permet avantageusement aux praticiens d'adapter leurs pratiques œnologiques visant à éviter l'altération du vin, en particulier concernant le sulfitage, et notamment de limiter l'usage du dioxyde de soufre en vinification aux cas pour lesquels cet usage s'avère vraiment utile.

L'échantillon sur lequel est mis en œuvre le procédé selon l'invention peut être un échantillon liquide, tel qu'une fraction d'un vin. Il peut autrement s'agir d'un échantillon ayant été prélevé à partir d'une surface telle qu'un sol, la paroi d'un matériel mis en œuvre pour la vinification, par exemple une cuve, etc., et déposé sur ou contenu dans un support solide ou semi-solide. De tels supports solides ou semi-solides peuvent notamment consister en des boites, lames de contact, le cas échéant gélosées, écouvillons, chiffonnettes, éponges, etc.

Selon des modes de mise en œuvre particuliers, le procédé selon l'invention répond en outre aux caractéristiques suivantes, mises en œuvre séparément ou en chacune de leurs combinaisons techniquement opérantes.

Tout marqueur moléculaire spécifique de telles levures peut être visé par l'étape de détection du procédé selon l'invention. Il est du ressort de l'homme du métier d'identifier les marqueurs moléculaires permettant de discriminer les levures de l'espèce *Brettanomyces bruxellensis* du groupe génétique des levures triploïdes œnologiques, de celles des groupes génétiques diploïdes œnologiques et triploïdes de bière, notamment sur la base de ces connaissances générales et des données publiées concernant ces levures, notamment des données génotypiques.

En particulier, il a été découvert par les présents inventeurs que la séquence protéique SEQ ID No: 1 (numéro d'accession Genbank : EIF47840.1) est spécifique des levures de l'espèce *Brettanomyces bruxellensis* du groupe des levures triploïdes œnologiques. La protéine présentant cette séquence, produite notamment par la souche AWRI1499 de *Brettanomyces bruxellensis,* est connue sous le nom de « putative histone acétyl transférase saga complex component ». La souche AWRI1499 est disponible dans la collection AWMCC (AWRI Wine Microorganism Culture Collection, Glen Osmond, Australie)

Ainsi, dans des modes de mise en œuvre particuliers de l'invention, l'étape de détection du procédé d'analyse comprend la détection, pour des levures contenues dans l'échantillon, d'un marqueur moléculaire associé à la protéine de séquence d'acides aminés SEQ ID No: 1.

Par marqueur moléculaire associé à la protéine, on entend que le marqueur moléculaire peut être constitué par la protéine elle-même, ou par tout fragment de cette dernière, notamment tout fragment de taille supérieure ou égale à 30 acides aminés, ou encore par tout acide nucléique associé à cette protéine, notamment tout segment du gène codant cette protéine, ou tout segment situé en amont ou en aval de ce gène, et contenant des séquences promotrices et/ou régulatrices de ce gène.

En particulier, le choix d'un marqueur moléculaire de nature nucléotidique s'avère tout à fait avantageux, car le procédé d'analyse selon l'invention permet alors de réaliser une évaluation particulièrement fiable de la capacité de résistance aux sulfites des levures de l'espèce *Brettanomyces bruxellensis* contenues dans l'échantillon, par analyse génétique de ces levures.

Le marqueur moléculaire, spécifique des levures de l'espèce *Brettanomyces bruxellensis* du groupe des levures triploïdes œnologiques, peut notamment être constitué par tout segment d'au moins 90 nucléotides successifs de la séquence nucléotidique de séquence SEQ ID No: 2 ou de toute séquence nucléotidique présentant avec cette séquence SEQ ID No: 2 un degré d'homologie supérieur ou égal à 90 % sur 80 % ou plus de la séquence. La séquence nucléotidique SEQ ID No: 2 inclut le gène codant la protéine « putative histone acetyl transferase saga complex component » mentionnée ci-avant, ainsi que les séquences contenant les séquences promotrices et régulatrices de ce gène, situées en amont et en aval de ce dernier, d'une taille d'environ 1 Kb chacune.

Ainsi, dans des modes de mise en œuvre particuliers de l'invention, l'étape de détection du procédé d'analyse comprend la détection, notamment à partir d'un extrait d'acides nucléiques obtenu à partir de levures contenues dans l'échantillon, d'un segment d'au moins 90 nucléotides successifs de la séquence nucléotidique de séquence SEQ ID No: 2 ou de toute séquence nucléotidique présentant avec cette séquence SEQ ID No: 2 un degré d'homologie supérieur ou égal à 90 % sur 80 % ou plus de la séquence.

Toute technique de détection d'un marqueur moléculaire classique en elle-même peut être mise en œuvre dans le cadre de l'invention.

Lorsque le marqueur moléculaire visé par l'étape de détection du procédé d'analyse selon l'invention est un peptide ou une protéine, cette étape de détection peut par exemple mettre en œuvre la technique connue sous le nom d'immunoabsorption enzymatique ELISA (pour l'anglais Enzyme-linked Immunosorbent Assay).

Lorsque ce marqueur moléculaire est une séquence nucléotidique, l'étape de détection du procédé d'analyse selon l'invention peut en particulier mettre en œuvre la technique de réaction de polymérisation en chaine (PCR).

Ainsi, dans des modes de mise en œuvre particuliers de l'invention, l'étape de détection comprend :
- l'extraction d'acides nucléiques de levures contenues dans l'échantillon,
- l'amplification, par réaction de polymérisation en chaine, d'un locus contenant le marqueur moléculaire spécifique des levures de l'espèce *Brettanomyces bruxellensis* du groupe des levures triploïdes œnologiques visé,
- et la détection dudit marqueur moléculaire dans l'ADN ainsi amplifié, par exemple par séparation des amplicons par électrophorèse, notamment en gel d'agarose ou capillaire, et analyse de taille.

A titre d'exemple, la réaction de polymérisation en chaine peut mettre en œuvre au moins une amorce nucléotidique dont la séquence nucléotidique contient une séquence choisie parmi les séquences SEQ ID No: 3 et SEQ ID No: 4. Elle met de préférence en œuvre un couple d'amorces nucléotidiques dont les séquences nucléotidiques contiennent respectivement les séquences SEQ ID No: 3 et SEQ ID No: 4. Lorsque le couple d'amorces nucléotidiques mis en œuvre présente les séquences respectives SEQ ID No: 3 et SEQ ID No: 4, il permet d'amplifier la séquence nucléotidique de séquence SEQ ID No: 7, de 263 paires de bases.

Le procédé d'analyse selon l'invention peut être mis en œuvre à partir d'acides nucléiques extraits aussi bien d'un mélange de levures contenues dans l'échantillon, que de colonies individuelles de levures isolées de cet échantillon.

Ainsi, dans des modes de mise en œuvre particuliers de l'invention, l'étape de détection comprend :
- l'isolement, à partir de l'échantillon, de colonies de levures de l'espèce *Brettanomyces bruxellensis* ; ceci peut par exemple être réalisé par étalement et culture des levures sur un milieu sélectif de l'espèce *Brettanomyces bruxellensis,* par exemple sur un milieu dit NS, pour *Non-Saccharomyces* ; de tels milieux sont bien connus de l'homme du métier ;
- l'extraction d'acides nucléiques, notamment de l'ADN génomique, de chacune de ces colonies,
- et la détection, dans chacun des extraits d'acides nucléiques ainsi obtenus, dudit marqueur moléculaire.

Cette détection peut notamment mettre en œuvre la technique de réaction de polymérisation en chaine, comme explicité ci-avant.

Un tel mode de mise en œuvre de l'invention s'avère particulièrement avantageux, notamment en ce qu'il permet, facilement et rapidement, de comptabiliser les colonies pour lesquelles ledit marqueur moléculaire peut être détecté, par rapport au nombre total de colonies analysées, et d'en déduire la proportion approximative de levures présentes dans l'échantillon qui sont résistantes aux sulfites. La connaissance d'une telle proportion approximative s'avère notamment utile pour les praticiens chargés de l'élaboration des vins, dans leur prise de décision de procéder ou non au sulfitage lors de la vinification. En effet, dans les cas où les levures de l'espèce *Brettanomyces bruxellensis* qui sont résistantes aux sulfites sont présentes en quantité majoritaire dans l'échantillon, dont le contenu en levures est représentatif de celui du vin, il pourra être décidé de ne pas avoir recours au sulfitage, qui serait de peu d'efficacité et qui serait en outre associé à un risque de renforcement de la résistance aux sulfites de l'espèce.

Dans des modes de mise en œuvre particuliers de l'invention, le procédé d'analyse comprend en outre une étape de vérification que les levures, pour lesquelles est mise en œuvre la détection du marqueur moléculaire spécifique des levures de l'espèce *Brettanomyces bruxellensis* du groupe des levures triploïdes œnologiques, appartiennent bien à l'espèce *Brettanomyces bruxellensis.* Cette étape peut être réalisée préalablement, ou de manière concomitante, à l'étape de détection de la présence éventuelle dans l'échantillon de levures de l'espèce *Brettanomyces bruxellensis* appartenant au groupe des levures triploïdes œnologiques. Elle peut notamment être mise en œuvre par détection d'une séquence nucléotidique spécifique de l'espèce *Brettanomyces bruxellensis,* par exemple par amplification par réaction de polymérisation en chaine au moyen d'amorces nucléotidiques appropriées, et analyse de la taille des amplicons obtenus, après séparation par électrophorèse par exemple.

A titre d'exemple, la réaction de polymérisation en chaine, pour la vérification de l'appartenance des levures à l'espèce *Brettanomyces bruxellensis,* peut mettre en œuvre au moins une amorce nucléotidique dont la séquence nucléotidique contient une séquence choisie parmi les séquences SEQ ID No: 5 et SEQ ID No: 6. Elle met de préférence en œuvre un couple d'amorces nucléotidiques de séquences nucléotidiques contenant respectivement les séquences SEQ ID No: 5 et SEQ ID No: 6. Le couple d'amorces de séquences respectives SEQ ID No: 5 et SEQ ID No: 6, qui est décrit dans la publication de Ibeas et al., 1996, dans Applied and Environmental Microbiology, 62:998-1003, comme spécifique de l'espèce *Brettanomyces bruxellensis,* permet d'amplifier pour cette espèce une séquence nucléotidique de 470 paires de bases.

A titre d'exemple, le procédé d'analyse selon l'invention peut comprendre la mise en œuvre d'une réaction de polymérisation en chaine, à partir d'ADN génomique extrait de levures contenues dans l'échantillon, au moyen de deux couples d'amorces nucléotidiques, dont un couple d'amorces spécifique des levures de l'espèce *Brettanomyces bruxellensis,* et un couple d'amorces nucléotidiques spécifique des levures de cette espèce appartenant au groupe génétique des levures triploïdes œnologiques.

Le premier couple d'amorces nucléotidiques peut notamment être le suivant :
Amorce Db1 : 5' AGAAGTTGAACGGCCGCATTTGCAC 3' (SEQ ID No: 5)
Amorce Db2 : 5' AGGATTGTTGACACTCTCGCCGAGG 3' (SEQ ID No: 6)

Le deuxième couple d'amorces nucléotidiques peut notamment être le suivant :
Amorce F1 : 5' TCTTCCTCCGATCCTTTATCA 3' (SEQ ID No: 3)
Amorce R1 : 5' TTGCACGATTTGTCAGAATCC 3' (SEQ ID No: 4)

Lorsque ces deux couples d'amorces nucléotidiques sont mis en œuvre simultanément, après séparation des amplicons par électrophorèse, par exemple en gel d'agarose ou capillaire, le procédé selon l'invention comprend l'analyse de la taille des fragments obtenus. L'amplification d'une bande à environ 470 paires de bases (pb) confirme l'appartenance à l'espèce *Brettanomyces bruxellensis.* L'amplification de cette seule bande à 470 pb indique en outre l'appartenance au groupe génétique des levures diploïdes ; l'amplification d'une bande supplémentaire à 356 pb indique l'appartenance au groupe génétique des levures triploïdes de bière ; et l'amplification d'une bande supplémentaire à 263 pb indique l'appartenance au groupe génétique des levures triploïdes œnologiques.

Le procédé selon l'invention permet ainsi avantageusement, en une seule analyse et de manière rapide, qui plus est au moyen de matériel couramment mis en œuvre dans les laboratoires d'analyse œnologiques, tels qu'un thermocycleur et un dispositif d'électrophorèse, de déterminer la capacité de résistance aux sulfites des levures de l'espèce *Brettanomyces bruxellensis* contenues dans l'échantillon, et notamment de la population majoritaire de telles levures.

Lorsque l'étape de détection et/ou l'étape de vérification du procédé d'analyse selon l'invention font appel à la technique de réaction de polymérisation en chaine, cette technique est mise en œuvre de manière classique en elle-même pour un homme du métier. Ainsi, elle peut être réalisée par exemple à partir de 50 à 500 ng d'ADN, au moyen de 1,5 à 3 µM de chaque amorce nucléotidique.

Dans des modes de réalisation particuliers de l'invention, l'étape de détection du procédé d'analyse comprend la détermination de la quantité de levures de l'espèce *Brettanomyces bruxellensis* appartenant au groupe génétique des levures triploïdes œnologiques présentes dans l'échantillon analysé. Cette détermination peut être mise en œuvre par toute méthode classique en elle-même pour l'homme du métier, notamment par réaction de polymérisation en chaine quantitative (qPCR), au moyen d'un couple d'amorces apte à amplifier le marqueur moléculaire spécifique des levures de l'espèce *Brettanomyces bruxellensis* du groupe génétique des levures triploïdes œnologiques, par exemple un couple d'amorces nucléotidiques de séquences contenant respectivement les séquences SEQ ID No: 3 et SEQ ID No: 4.

Le procédé d'analyse selon l'invention peut en outre comprendre une étape de détermination de la quantité totale de levures à l'espèce *Brettanomyces bruxellensis* présentes dans l'échantillon analysé, notamment également par qPCR.

Dans de tels modes de mise en œuvre,le procédé selon l'invention permet avantageusement de quantifier précisément, d'une part la population totale de levures de l'espèce *Brettanomyces bruxellensis* présente dans l'échantillon, et d'autre part la proportion de levures de l'espèce *Brettanomyces bruxellensis* qui sont résistantes aux sulfites.

Selon un autre aspect, la présente invention concerne l'utilisation, pour l'analyse de la présence éventuelle, dans un échantillon, de levures de l'espèce *Brettanomyces bruxellensis* résistantes aux sulfites, d'un couple d'amorces nucléotidiques apte à amplifier, par réaction de polymérisation en chaine, une séquence nucléotidique spécifique des levures de l'espèce *Brettanomyces bruxellensis* appartenant au groupe génétique des levures triploïdes œnologiques.

Ce couple d'amorces nucléotidiques peut notamment être apte à amplifier un segment d'au moins 90 nucléotides successifs de la séquence nucléotidique de séquence SEQ ID No: 2 ou de toute séquence nucléotidique présentant avec cette séquence SEQ ID No: 2 un degré d'homologie supérieur ou égal à 90 % sur 80 % ou plus de la séquence.

Ce couple d'amorces nucléotidiques peut notamment être le suivant :
Amorce F1 : 5' TCTTCCTCCGATCCTTTATCA 3' (SEQ ID No: 3)
Amorce R1 : 5' TTGCACGATTTGTCAGAATCC 3' (SEQ ID No: 4)
ou consister en un couple d'amorces de séquences nucléotidiques contenant respectivement ces séquences SEQ ID No: 3 et SEQ ID No: 4.

L'invention concerne également l'utilisation d'un kit tel que défini dans la revendication 13, pour la mise en œuvre d'un procédé d'analyse selon l'invention.

Un autre aspect de la présente invention concerne un kit tel que défini dans la revendication 14, pour la mise en œuvre d'un procédé d'analyse selon l'invention, répondant à l'une ou plusieurs des caractéristiques ci-avant. Ce kit comprend :
- un couple d'amorces nucléotidiques pour l'amplification, par réaction de polymérisation en chaine, d'une séquence nucléotidique spécifique des levures de l'espèce *Brettanomyces bruxellensis* appartenant au groupe génétique des levures triploïdes œnologiques ;
- et un couple d'amorces nucléotidiques pour l'amplification, par réaction de polymérisation en chaine, d'une séquence nucléotidique témoignant de l'appartenance d'une levure à l'espèce *Brettanomyces bruxellensis,* spécifique de cette espèce.

En particulier, le kit selon l'invention comporte :
- un couple d'amorces nucléotidiques pour l'amplification, par réaction de polymérisation en chaine, d'un segment d'au moins 90 nucléotides successifs de la séquence nucléotidique de séquence SEQ ID No: 2 ou de toute séquence nucléotidique présentant avec cette séquence SEQ ID No: 2 un degré d'homologie supérieur ou égal à 90 % sur 80 % ou plus de la séquence, par exemple un couple d'amorces nucléotidiques de séquences nucléotidiques contenant respectivement les séquences SEQ ID No: 3 et SEQ ID No: 4 ;
- et un couple d'amorces nucléotidiques de séquences nucléotidiques contenant respectivement les séquences SEQ ID No: 5 et SEQ ID No: 6.

Le kit selon l'invention peut également comporter au moins un, en particulier plusieurs, et par exemple la totalité, des composants suivants :
- un réactif de réaction de polymérisation en chaine, tel qu'une Taq polymérase, le cas échéant associée à un tampon adapté,
- un réactif pour qPCR, tel qu'un réactif SYBR® Green ou une sonde moléculaire marquée pour système TaqMan®,
- un témoin de taille moléculaire d'acides nucléiques, notamment pour analyse par électrophorèse,
- un réactif d'extraction d'acides nucléiques de levures,
- et/ou des instructions de mise en œuvre des étapes d'un procédé selon la présente invention.

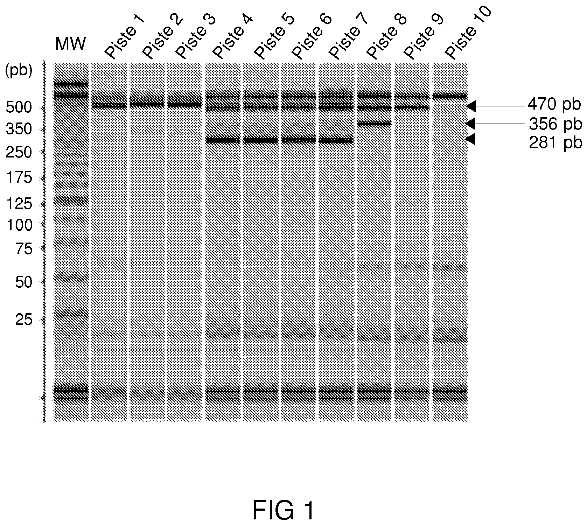

Les caractéristiques et avantages du procédé selon l'invention apparaîtront plus clairement à la lumière des exemples de mise en œuvre ci-après, fournis à simple titre illustratif et nullement limitatifs de l'invention, avec l'appui de la figure 1, qui représente un gel d'électrophorèse réalisé sur des produits d'amplification par PCR, selon un protocole conforme à l'invention, pour : pistes 1 à 6, des clones de levures de l'espèce *Brettanomyces bruxellensis* obtenus à partir d'un échantillon de vin rouge élevé en barriques ; piste 7, un témoin connu pour son appartenance au groupe génétique des levures triploïdes œnologiques ; piste 8, un témoin connu pour son appartenance au groupe génétique des levures triploïdes de bière ; piste 9, un témoin connu pour son appartenance au groupe génétique des levures diploïdes œnologiques; piste 10, un témoin négatif; la première piste correspondant au marqueur de poids moléculaire (MW).

### EXEMPLE 1

Il est mis en œuvre le procédé suivant, pour déterminer, pour 33 souches différentes, leur répartition dans les trois groupes génétiques : diploïde œnologique, triploïde œnologique et triploïde de bière.

Pour chacune de ces 33 souches, des cellules sont prélevées à partir d'un étalement sur boite de Petri, diluées individuellement dans une solution de NaOH 20mM à une concentration d'environ 10⁸ cellules/mL, puis la solution obtenue est chauffée pendant 10 min à 94 °C. Cette matrice, qui contient l'ADN, est utilisée pour l'étape subséquente d'amplification PCR.

L'étape d'amplification par PCR est réalisée conformément à l'invention, en utilisant le mélange suivant :
- 3 µM d'amorce M13-F1 (SEQ ID No: 8 : ACGACGTTGTAAAACGACTCTTCCTCCGATCCTTTATCA)
- 3 µM d'amorce R1 (SEQ ID No: 4 : TTGCACGATTTGTCAGAATCC)
- 1,5 µM d'amorce Db1 (SEQ ID No: 5 : AGAAGTTGAACGGCCGCATTTGCAC)
- 1,5 µM d'amorce Db2 (SEQ ID No: 6 : AGGATTGTTGACACTCTCGCGGAGG)
- Taq polymerase + tampon adapté (1X Taq-&GO® de MP Biomedicals)
- 1µl de matrice d'ADN en solution de NaOH (comportant entre 50 et 500 ng d'ADN).

L'amorce M13-F1 contient la séquence SEQ ID No: 3, ainsi que, en son extrémité 5', la séquence d'une queue M13 de 18 nucléotides.

Le programme PCR est le suivant :
- 5 min à 95 °C,
- 35 cycles de (30 s à 95 °C ; 30 s à 57 °C ; 1 minà 72 °C),
- 10 min à 72°C.

A l'issue de l'étape d'amplification par PCR, les amplicons sont séparés par électrophorèse en gel d'agarose à 2 %.

A titre de témoin, il est également réalisé une réaction PCR avec le seul couple d'amorces Db1/Db2. L'apparition sur le gel d'agarose d'une seule bande à environ 470 pb, pour l'ensemble des souches testées, confirme que ces souches appartiennent bien toutes à l'espèce *Brettanomyces bruxellensis.*

Les différentes souches de *Brettanomyces bruxellensis* sont classées en trois groupes : les souches pour lesquelles apparait, après PCR avec les deux couples d'amorces ci-avant, la seule bande à environ 470 pb, sont classées comme appartenant au groupe des levures diploïdes œnologiques ; les souches pour lesquelles apparait, en plus de cette bande à environ 470 pb, une bande supplémentaire à 356 pb, sont classées comme appartenant au groupe des levures triploïdes de bière ; et les souches pour lesquelles apparait, en plus de la bande à environ 470 pb, une bande supplémentaire à 281 pb, sont classées comme appartenant au groupe des levures triploïdes œnologiques.

Les 33 souches testées sont ainsi réparties en : 17 souches du groupe génétique des levures diploïdes œnologiques, 8 souches du groupe génétique des levures triploïdes œnologiques et 8 souches du groupe génétique des levures triploïdes de bière.

La corrélation entre la triploïdie des souches de *Brettanomyces bruxellensis* et leur sensibilité/résistance aux sulfites est confirmée de la façon suivante.

Les 33 souches de *Brettanomyces bruxellensis* sont cultivées dans un milieu en absence ou en présence de différentes concentrations de dioxyde de soufre SO₂ dans le milieu de culture (0 mg/L, 0,2 mg/L, 0,4 mg/L et 0,6 mg/L).

Après 14 jours de culture, la capacité des souches à croître dans les différents milieux est évaluée. Les résultats obtenus sont indiqués dans le tableau 1 ci-dessous.

**Tableau 1 - Evaluation de la capacité des souches de chaque groupe génétique à croître en présence de dioxyde de soufre**

| | Nombre de souches capables de croître | | | |
|---|---|---|---|---|
| Concentration en SO₂ actif dans le milieu de culture (mg/L) | 0 | 0,2 | 0,4 | 0,6 |
| Diploïde œnologique (17 souches) | 17 | 6 | 1 | 0 |
| Triploïde bière (8 souches) | 8 | 3 | 0 | 0 |
| Triploïde œnologique (8 souches) | 8 | 8 | 8 | 4 |

La relation entre le groupe génétique et la capacité de résistance aux sulfites est confirmée pour toutes les souches testées.

### EXEMPLE 2

Un procédé conforme à l'invention est mis en œuvre à partir d'un échantillon d'un grand vin du Châteaux X en élevage.

Le prélèvement de cet échantillon avait été effectué en octobre 2014 sur barrique.

Plusieurs volumes de cet échantillon (10 ml et 100 µl) sont étalés sur milieu sélectif dit NS (pour « *Non-Saccharomyces* »), contenant : extrait de levure (10 g/l), BactoPeptone (10 g/l), glucose (20 g/l), agar (20g/l), chloramphénicol (0,1 mg/ml), biphényle (0,15 mg/ml), actidione (0,5 mg/ml).

Après 7 jours d'incubation à 30 °C, on observe, pour l'échantillon « 10 ml », la présence d'un tapis cellulaire, et, pour l'échantillon « 100 µl », des clones isolés.

Six de ces clones isolés sont prélevés, dilués individuellement dans une solution de NaOH 20 mM à une concentration d'environ 10⁸ cellules/mL, puis la solution est chauffée pendant 10 min à 94 °C.

Chaque matrice d'ADN ainsi obtenue est soumise à une étape d'amplification par PCR, selon les conditions opératoires décrites dans l'Exemple 1 ci-avant.

A l'issue de cette étape, chaque solution obtenue est diluée au ½, et analysée par électrophorèse en capillaire (MultiNA, Shimadzu).

Des souches témoins connues pour leur appartenance à un groupe génétique donné sont soumises au même procédé : témoin triploïde œnologique, témoin triploïde bière, témoin diploïde œnologique, de même qu'un témoin négatif, dans lequel la matrice d'ADN est remplacée par de l'eau distillée.

Le gel d'électrophorèse obtenu est montré sur la figure 1.

Comme on peut l'observer, sur les 6 clones testés, 3 clones appartiennent au groupe diploïde œnologique (sensible aux sulfites) (pistes 1 à 3) et 3 clones appartiennent au groupe triploïde œnologique (résistant aux sulfites) (pistes 4 à 6). 50 % des souches de *B. bruxellensis* sont ainsi résistantes aux sulfites, et 50 % y sont sensibles.

### EXEMPLE 3 - Test quantitatif

Un procédé conforme à l'invention est mis en œuvre à partir de l'échantillon de vin de l'Exemple 2.

Une extraction d'ADN total de l'échantillon est tout d'abord réalisée à l'aide du kit commercial VlNEO® Extract DNA Kit de Biorad.

La matrice d'ADN ainsi obtenue est soumise à une PCR quantitative. A cet effet, il est mis en œuvre le mélange suivant :
- 1 à 10 µM de chacune des amorces M13-F1 et R1, et le cas échéant des amorces Db1 et Db2,
- 1X Taq polymerase + tampon adapté + SYBR® Green PCR Supermix de Bio-Rad
- 50-100 ng d'ADN extrait.

Une gamme étalon est également réalisée, à partir d'un échantillon de concentration connue en une souche de *Brettanomyces bruxellensis* connue pour appartenir au groupe triploïde œnologique.

La quantité de levures de l'espèce *Brettanomyces bruxellensis* appartenant au groupe triploïde œnologique, donc résistantes aux sulfites, présentes dans l'échantillon de vin initial, est ainsi déterminée.

En parallèle, la quantité totale de levures de l'espèce *Brettanomyces bruxellensis* présentes dans l'échantillon de vin peut également être déterminée, par exemple, de manière classique en elle-même, au moyen du kit VlNEO® Brettanomytest Kit PCR de la société Biorad.

## Revendications

1. Procédé d'analyse d'un échantillon pour la présence éventuelle de levures de l'espèce *Brettanomyces bruxellensis* résistantes aux sulfites, **caractérisé en ce que** :
- on procède à une étape de détection de la présence éventuelle, dans ledit échantillon, de levures de l'espèce *Brettanomyces bruxellensis* appartenant au groupe génétique des levures triploïdes œnologiques, comprenant la détection, pour des levures contenues dans ledit échantillon, d'un marqueur moléculaire spécifique des levures de l'espèce *Brettanomyces bruxellensis* du groupe génétique des levures triploïdes œnologiques,
- et, si on détecte une telle présence, on déduit que ledit échantillon contient des levures de l'espèce *Brettanomyces bruxellensis* résistantes aux sulfites.

2. Procédé d'analyse selon la revendication 1, selon lequel ledit marqueur moléculaire est associé à la protéine de séquence d'acides aminés SEQ ID No: 1.

3. Procédé d'analyse selon l'une quelconque des revendications 1 à 2, selon lequel ledit marqueur moléculaire est constitué par tout segment d'au moins 90 nucléotides successifs de la séquence nucléotidique de séquence SEQ ID No: 2 ou de toute séquence nucléotidique présentant avec ladite séquence SEQ ID No: 2 un degré d'homologie supérieur ou égal à 90 % sur 80 % ou plus de la séquence.

4. Procédé d'analyse selon l'une quelconque des revendications 1 à 3, selon lequel ladite étape de détection comprend :
- l'extraction d'acides nucléiques de levures contenues dans ledit échantillon,
- l'amplification d'un locus contenant ledit marqueur moléculaire par réaction de polymérisation en chaine,
- et la détection dudit marqueur moléculaire dans l'ADN ainsi amplifié.

5. Procédé d'analyse selon la revendication 4, selon lequel la réaction de polymérisation en chaine met en œuvre au moins une amorce nucléotidique de séquence contenant une séquence choisie parmi les séquences SEQ ID No: 3 et SEQ ID No: 4.

6. Procédé selon l'une quelconque des revendications 1 à 5, selon lequel ladite étape de détection comprend :
- l'isolement, à partir dudit échantillon, de colonies de levures de l'espèce *Brettanomyces bruxellensis,*
- l'extraction d'acides nucléiques de chacune de ces colonies,
- et la détection, dans chacun des extraits d'acides nucléiques ainsi obtenus, dudit marqueur moléculaire.

7. Procédé d'analyse selon l'une quelconque des revendications 1 à 6, comprenant une étape de vérification que les levures pour lesquelles est mise en œuvre la détection dudit marqueur moléculaire appartiennent à l'espèce *Brettanomyces bruxellensis.*

8. Procédé d'analyse selon l'une quelconque des revendications 1 à 7, selon lequel ladite étape de détection comprend la détermination de la quantité de levures de l'espèce *Brettanomyces bruxellensis* appartenant au groupe génétique des levures triploïdes œnologiques présentes dans ledit échantillon.

9. Procédé d'analyse selon la revendication 8, selon lequel ladite détermination est mise en œuvre par réaction de polymérisation en chaine quantitative.

10. Procédé d'analyse selon l'une quelconque des revendications 1 à 9, comprenant une étape de détermination de la quantité de levures à l'espèce *Brettanomyces bruxellensis* présentes dans ledit échantillon.

11. Utilisation d'un couple d'amorces nucléotidiques apte à amplifier, par réaction de polymérisation en chaine, une séquence nucléotidique spécifique des levures de l'espèce *Brettanomyces bruxellensis* appartenant au groupe génétique des levures triploïdes œnologiques, pour l'analyse de la présence éventuelle, dans un échantillon, de levures de l'espèce *Brettanomyces bruxellensis* résistantes aux sulfites.

12. Utilisation selon la revendication 11, selon laquelle ledit couple d'amorces nucléotidiques est apte à amplifier un segment d'au moins 90 nucléotides successifs de la séquence nucléotidique de séquence SEQ ID No: 2 ou de toute séquence nucléotidique présentant avec ladite séquence SEQ ID No: 2 un degré d'homologie supérieur ou égal à 90 % sur 80 % ou plus de la séquence.

13. Utilisation pour la mise en œuvre d'un procédé d'analyse selon l'une quelconque des revendications 1 à 10, d'un kit comprenant :
- un couple d'amorces nucléotidiques pour l'amplification, par réaction de polymérisation en chaine, d'une séquence nucléotidique spécifique des levures de l'espèce *Brettanomyces bruxellensis* appartenant au groupe génétique des levures triploïdes œnologiques ;
- et un couple d'amorces nucléotidiques pour l'amplification, par réaction de polymérisation en chaine, d'une séquence nucléotidique témoignant de l'appartenance d'une levure à l'espèce *Brettanomyces bruxellensis.*

14. Kit pour la mise en œuvre d'un procédé d'analyse selon l'une quelconque des revendications 1 à 10, **caractérisé en ce qu'**il comprend :
- un couple d'amorces nucléotidiques pour l'amplification, par réaction de polymérisation en chaine, d'un segment d'au moins 90 nucléotides successifs de la séquence nucléotidique de séquence SEQ ID No: 2 ou de toute séquence nucléotidique présentant avec ladite séquence SEQ ID No: 2 un degré d'homologie supérieur ou égal à 90 % sur 80 % ou plus de la séquence, ledit segment étant spécifique des levures de l'espèce *Brettanomyces bruxellensis* appartenant au groupe génétique des levures triploïdes œnologiques ;
- et un couple d'amorces nucléotidiques de séquences contenant respectivement les séquences SEQ ID No: 5 et SEQ ID No: 6, permettant l'amplification, par réaction de polymérisation en chaine, d'une séquence nucléotidique témoignant de l'appartenance d'une levure à l'espèce *Brettanomyces bruxellensis.*

## Patentansprüche

1. Verfahren zur Analyse einer Probe für das eventuelle Vorhandensein von sulfitresistenten Hefen der Gattung *Brettanomyces bruxellensis,* **dadurch gekennzeichnet, dass**:
- ein Detektionsschritt bezüglich des eventuellen Vorhandenseins in der Probe von Hefen der Gattung *Brettanomyces bruxellensis* durchgeführt wird, die zur genetischen Gruppe der önologischen triploiden Hefen gehören, umfassend das Detektieren, für die in der Probe enthaltenen Hefen, eines spezifischen molekularen Markers der Hefen der Gattung *Brettanomyces bruxellensis* aus der genetischen Gruppe der önologischen triploiden Hefen,
- und, bei Detektion eines derartigen Vorhandenseins, das Schlussfolgern, dass die Probe sulfitresistente Hefen der Gattung *Brettanomyces bruxellensis* enthält.

2. Analyseverfahren nach Anspruch 1, wobei der molekulare Marker dem Protein mit der Aminosäuresequenz SEQ ID NO: 1 zugeordnet ist.

3. Analyseverfahren nach einem der Ansprüche 1 bis 2, wobei der molekulare Marker von jedem Segment mit mindestens 90 aufeinanderfolgenden Nukleotiden der Nukleotidsequenz mit der Sequenz SEQ ID NO: 2 oder jeder Nukleotidsequenz gebildet ist, die mit der Sequenz SEQ ID NO: 2 einen Übereinstimmungsgrad von größer oder gleich 90 % bei über 80 % oder mehr der Sequenz aufweist.

4. Analyseverfahren nach einem der Ansprüche 1 bis 3, wobei der Detektionsschritt umfasst:
- das Extrahieren von Nukleinsäuren von Hefen, die in der Probe enthalten sind,
- das Amplifizieren eines Lokus, der den molekularen Marker enthält, durch Polymerisationskettenreaktion,
- und das Detektieren des molekularen Markers in der derart amplifizierten DNA.

5. Analyseverfahren nach Anspruch 4, wobei die Polymerisationskettenreaktion mindestens einen Nukleotidprimer mit der Sequenz umsetzt, die eine Sequenz enthält, die aus den Sequenzen SEQ ID NO: 3 und SEQ ID NO: 4 ausgewählt ist.

6. Verfahren nach einem der Ansprüche 1 bis 5, wobei der Detektionsschritt umfasst:
- das Isolieren, auf der Basis der Probe, von Hefekolonien der Gattung *Brettanomyces bruxellensis,*
- das Extrahieren von Nukleinsäuren von jeder dieser Kolonien,
- und das Detektieren, in jedem der derart erhaltenen Nukleinsäureextrakte, des molekularen Markers.

7. Analyseverfahren nach einem der Ansprüche 1 bis 6, umfassend einen Schritt des Überprüfens, dass die Hefen, für die die Detektion des molekularen Markers umgesetzt wurde, zu der Gattung *Brettanomyces bruxellensis* gehören.

8. Analyseverfahren nach einem der Ansprüche 1 bis 7, wobei der Detektionsschritt das Bestimmen der Menge an Hefen der Gattung *Brettanomyces bruxellensis* umfasst, die zur genetischen Gruppe der önologischen triploiden Hefen gehören, die in der Probe vorhanden sind.

9. Analyseverfahren nach Anspruch 8, wobei die Bestimmung durch quantitative Polymerisationskettenreaktion durchgeführt wird.

10. Analyseverfahren nach einem der Ansprüche 1 bis 9, umfassend einen Bestimmungsschritt der Menge an Hefen der Gattung *Brettanomyces bruxellensis,* die in der Probe vorhanden sind.

11. Verwendung eines Nukleotidprimerpaars, das imstande ist, durch Polymerisationskettenreaktion eine spezifische Nukleotidsequenz der Hefen der Gattung *Brettanomyces bruxellensis* zu amplifizieren, die zur genetischen Gruppe der önologischen triploiden Hefen gehören, für die Analyse des eventuellen Vorhandenseins in einer Probe von sulfitresistenten Hefen der Gattung *Brettanomyces bruxellensis.*

12. Verwendung nach Anspruch 11, wobei das Nukleotidprimerpaar imstande ist, ein Segment mit mindestens 90 aufeinanderfolgenden Nukleotiden der Nukleotidsequenz mit der Sequenz SEQ ID NO: 2 oder jeder anderen Nukleotidsequenz zu amplifizieren, die mit der Sequenz SEQ ID NO: 2 einen Übereinstimmungsgrad von größer oder gleich 90 % bei über 80 % oder mehr der Sequenz aufweist.

13. Verwendung eines Kits für die Durchführung eines Analyseverfahrens nach einem der Ansprüche 1 bis 10, umfassend:
- ein Nukleotidprimerpaar für die Amplifizierung durch Polymerisationskettenreaktion einer spezifischen Nukleotidsequenz der Hefen der Gattung *Brettanomyces bruxellensis,* die zur genetischen Gruppe der önologischen triploiden Hefen gehören;
- und ein Nukleotidprimerpaar für die Amplifizierung durch Polymerisationskettenreaktion einer Nukleotidsequenz, die die Zugehörigkeit einer Hefe zur Gattung *Brettanomyces bruxellensis* nachweist.

14. Kit für die Durchführung eines Analyseverfahrens nach einem der Ansprüche 1 bis 10, **dadurch gekennzeichnet, dass** es umfasst:
- ein Nukleotidprimerpaar für die Amplifizierung durch Polymerisationskettenreaktion eines Segments mit mindestens 90 aufeinanderfolgenden Nukleotiden der Nukleotidsequenz mit der Sequenz SEQ ID NO: 2 oder jeder Nukleotidsequenz, die mit der Sequenz SEQ ID NO: 2 einen Übereinstimmungsgrad von größer oder gleich 90 % bei über 80 % oder mehr der Sequenz aufweist, wobei das Segment für die Hefen der Gattung *Brettanomyces bruxellensis* spezifisch ist, die zur genetischen Gruppe der önologischen triploiden Hefen gehören;
- und ein Nukleotidprimerpaar mit Sequenzen, die jeweils die Sequenzen SEQ ID NO: 5 und SEQ ID NO: 6 enthalten, die die Amplifizierung durch Polymerisationskettenreaktion einer Nukleotidsequenz erlauben, die die Zugehörigkeit einer Hefe zur Gattung *Brettanomyces bruxellensis* nachweist.

## Claims

1. Analysis method for analyzing a sample for the possible presence of sulphite-resistant yeasts of the *Brettanomyces bruxellensis* species, **characterized in that** it comprises:
- a step of detection of the possible presence, in said sample, of yeasts of the *Brettanomyces bruxellensis* species belonging to the genetic group of oenological triploid yeasts, comprising the detection, for yeasts contained in said sample, of a molecular marker specific to the yeasts of the *Brettanomyces bruxellensis* species of the genetic group of oenological triploid yeasts,
- and, if such a presence is detected, the deduction that said sample contains yeasts of the *Brettanomyces bruxellensis* species resistant to sulphites.

2. Analysis method according to claim 1, wherein said molecular marker is associated with the protein having the amino acid sequence SEQ ID no. 1.

3. Analysis method according to any of claims 1 to 2, wherein said molecular marker is constituted of any segment of at least 90 successive nucleotides of the nucleotide sequence of sequence SEQ ID no. 2 or of any nucleotide sequence having with said sequence SEQ ID no. 2 a degree of homology higher or equal to 90 % on 80 % or more of the sequence.

4. Analysis method according to any of claims 1 to 3, wherein said detection step comprises:
- the extraction of nucleic acids from yeasts contained in said sample,
- the amplification of a locus containing said molecular marker by polymerase chain reaction,
- and the detection of said molecular marker in the DNA thus amplified.

5. Analysis method according to claim 4, wherein the polymerase chain reaction implements at least a nucleotide primer the sequence of which contains a sequence chosen among sequences SEQ ID no. 3 and SEQ ID no 4.

6. Analysis method according to any of claims 1 to 5, wherein said detection step comprises:
- the isolation, from said sample, of colonies of yeasts of the *Brettanomyces bruxellensis* species,
- the extraction of nucleic acids from each of these colonies,
- and the detection, in each of the nucleic acid extracts thus obtained, of said molecular marker.

7. Analysis method according to any of claims 1 to 6, comprising a step of verifying that the yeasts, for which the detection of said molecular marker is performed, belong to the *Brettanomyces bruxellensis* species.

8. Analysis method according to any of claims 1 to 7, wherein said detection step comprises a determination step for determining the quantity of yeasts of the *Brettanomyces bruxellensis* species belonging to the genetic group of oenological triploid yeasts present in said sample.

9. Analysis method according to claim 8, wherein said determination step is realized by quantitative polymerase chain reaction.

10. Analysis method according to any of claims 1 to 9, comprising a step of determining the quantity of yeasts of the *Brettanomyces bruxellensis* species present in said sample.

11. Use of a pair of nucleotide primers capable of amplifying, by polymerase chain reaction, a nucleotide sequence specific to the yeasts of the *Brettanomyces bruxellensis* species belonging to the genetic group of oenological triploid yeasts, for analyzing the possible presence, in a sample, of sulphite-resistant yeasts of the *Brettanomyces bruxellensis* species.

12. Use according to claim 11, wherein said pair of nucleotide primers is capable of amplifying a segment of at least 90 successive nucleotides of the nucleotide sequence of sequence SEQ ID no. 2 or of any nucleotide sequence having with said sequence SEQ ID no. 2 a degree of homology higher or equal to 90 % on 80 % or more of the sequence.

13. Use for implementing an analysis method according to any of claims 1 to 10, of a kit comprising:
- a pair of nucleotide primers for the amplification, by polymerase chain reaction, of a nucleotide sequence specific to yeasts of the *Brettanomyces bruxellensis* species belonging to the genetic group of oenological triploid yeasts;
- and a pair of nucleotide primers for the amplification, by polymerase chain reaction, of a nucleotide sequence evidencing the belonging of a yeast to the *Brettanomyces bruxellensis* species.

14. Kit for implementing an analysis method according to any of claims 1 to 10, **characterized in that** it comprises:
- a pair of nucleotide primers for the amplification, by polymerase chain reaction, of a segment of at least 90 successive nucleotides of the nucleotide sequence of sequence SEQ ID no. 2 or of any nucleotide sequence having with said sequence SEQ ID no. 2 a degree of homology higher or equal to 90 % on 80 % or more of the sequence, said segment being specific of the yeasts of the *Brettanomyces bruxellensis* species belonging to the genetic group of oenological triploid yeasts;
- and a pair of nucleotide primers of sequences containing respectively sequence SEQ ID no. 5 and sequence SEQ ID no. 6, enabling the amplification, by polymerase chain reaction, of a nucleotide sequence evidencing the belonging of a yeast to the *Brettanomyces bruxellensis* species.
